# EUROPEAN PATENT APPLICATION

(11) **EP 3 037 052 A1**
(43) Date of publication of application: **29.06.2016**
(21) Application number: 14838264.1
(22) Date of filing: 11.03.2014
(51) Int. Cl.: A61B 17/34, A61B 18/12

(54) **TROCAR**

(30) Priority: 22.08.2013 JP 2013172474
(71) Applicant: Olympus Corporation, Shibuya-ku Tokyo 151-0072 (JP)
(72) Inventor: MATSUI, Akira, Tokyo 151-0072 (JP)
(74) Representative: Schicker, Silvia
(86) International application number: PCT/JP2014/056339
(87) International publication number: WO 2015/025547

(57) **Abstract**

A trocar 10 includes: a housing 14; an insertion tube 14H that is integral with the housing 14 and is inserted into a subject 9; a power transmission coil 11 that is disposed inside of the housing 14 and generates AC magnetic field to be applied to an insertion hole 10H into which a treatment tool 30 is to be inserted; a heat dissipation member 12 that transmits heat generated from the power transmission coil 11; and a heat insulation member 13 disposed on a side of the subject with reference to the heat dissipation member 12 and having lower thermal conductivity than the heat dissipation member 12.

## Description

### Technical Field

The present invention relates to a trocar to feed power to a treatment tool wirelessly.

### Background Art

A trocar is integrally combined with an inner needle having a sharp puncture needle at the forward end, and the inner needle in such a state is punctured through a body wall of a patient so as to be inserted into the abdominal cavity. After being inserted into the abdominal cavity, the inner needle is removed so as to leave the trocar at the body wall, and then the trocar is used as a guide tube for a treatment tool that is for treatment in the abdominal cavity.

Some treatment tools inserted into a trocar are connected to a cable to receive power required for the treatment. Such a cable hinders the manipulation by an operator during operation and degrades the operability.

To solve this problem, JP H11-128242 A discloses a technique of supplying power from a power-transmission coil of a trocar to a power-reception coil of a treatment tool that is inserted into the trocar.

An energy treatment tool for surgical operation, such as a electrosurgical knife or an ultrasonic knife, however, requires relatively large power of a few tens W to hundreds W for treatment, and so the power-transmission coil may generate heat and temperature of the trocar housing in contact with the patient may rise.

### Summary of the Invention

### Problems to be solved by the Invention

An embodiment of the present invention aims to provide a trocar capable of suppressing temperature rise at the housing.

### Means for solving the Problems

A trocar that is one embodiment includes: a housing; an insertion tube that is integral with the housing and is inserted into a subject; a power transmission coil that is disposed inside of the housing and generates AC magnetic field to be applied to an insertion hole into which a treatment tool is to be inserted; a heat dissipation member that transmits heat generated from the power transmission coil; and a heat insulation member disposed on a side of the subject with reference to the heat dissipation member and having lower thermal conductivity than the heat dissipation member.

### Advantageous Effect of the Invention

An embodiment of the present invention can provide a trocar capable of suppressing temperature rise at the housing.

### Brief Description of the Drawings

FIG. 1 schematically illustrates the usage state of an operating system including a trocar of a first embodiment.
FIG. 2 illustrates a circuit configuration of the operating system including the trocar of the first embodiment.
FIG. 3 is a partial cross sectional view of the trocar of the first embodiment.
FIG. 4 is a perspective view of a heat dissipation member and a heat insulation member of the trocar of the first embodiment.
FIG. 5A is a cross sectional view of a heat dissipation member and a power transmission coil of a trocar of the first embodiment.
FIG. 5B is a cross sectional view of a heat dissipation member and a power transmission coil of a trocar of the first embodiment.
FIG. 5C is a cross sectional view of a heat dissipation member and a power transmission coil of a trocar of the first embodiment.
FIG. 6A is a perspective view of a heat dissipation member as a modification example of the first embodiment.
FIG. 6B is a perspective view of a heat dissipation member as another modification example of the first embodiment.
FIG. 7 is an exploded view of a heat dissipation member as a modification example of the first embodiment.
FIG. 8 schematically illustrates the usage state of an operating system including a trocar of a second embodiment.
FIG. 9 illustrates a circuit configuration of the operating system including the trocar of the second embodiment.
FIG. 10 illustrates the configuration to describe a flow channel for gas in the operating system including the trocar of the second embodiment.

### Mode for carrying out the Invention

### <First Embodiment>

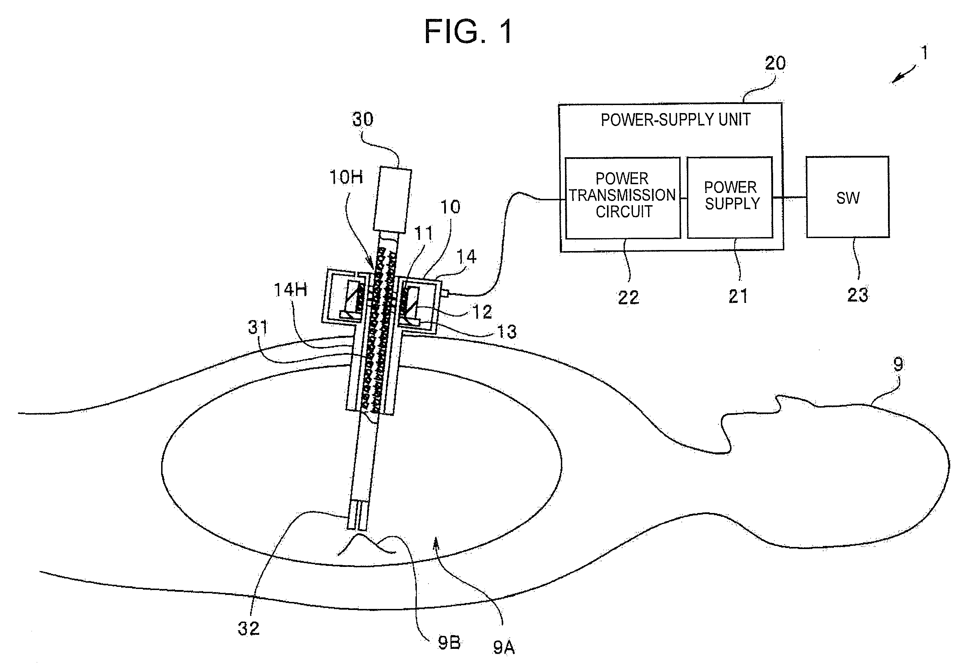

Referring firstly to FIGs. 1 to 4, the following describes an operating system 1 including a trocar 10 of a first embodiment. As illustrated in FIG. 1, the operating system 1 includes the trocar 10, a power-supply unit 20, a treatment tool 30, and a switch 23. Although the operating system 1 allows an endoscope or the like also to be inserted into an abdominal cavity 9A of a subject 9 via another trocar, the descriptions thereof are omitted.

The trocar 10 of the present embodiment includes a housing 14, an insertion tube 14H, a power transmission coil 11, a heat dissipation member 12, and a heat insulation member 13. The long and thin insertion tube 14H that is extended from a lower part of the housing 14 is inserted into the subject 9. The housing 14 and the insertion tube 14H are made of the same material and are configured integrally. The housing 14 has an insertion hole 10H at the center, through which the treatment tool 30 is to be inserted. The insertion hole 10H is a through hole that is extended to the forward end of the insertion tube 14H.

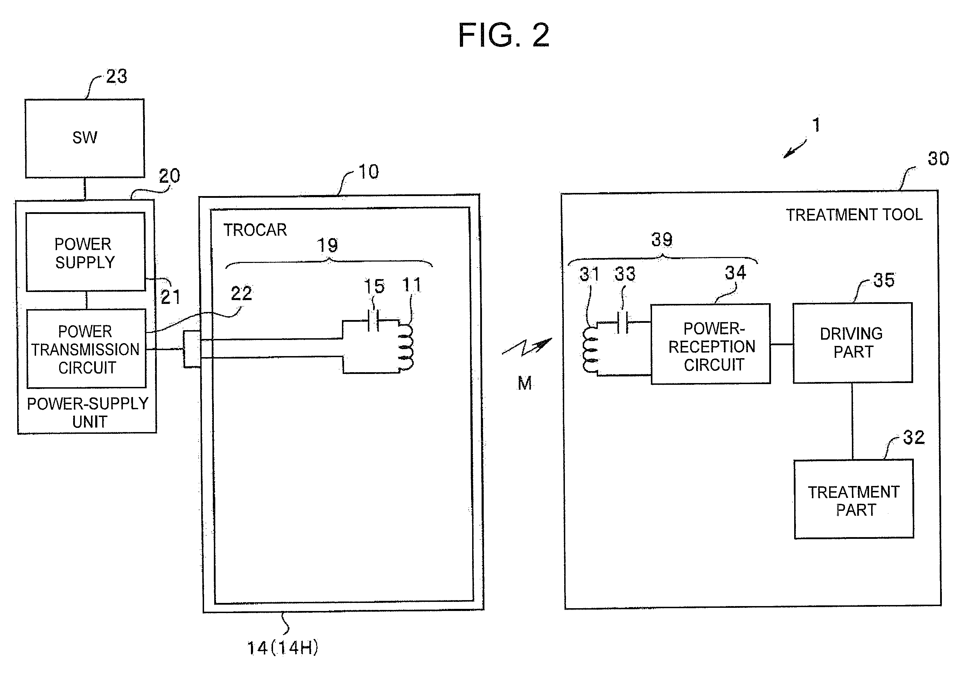

The treatment tool 30 is inserted into the abdominal cavity 9A through the insertion hole 10H. The treatment tool 30 of the present embodiment is a bipolar electrosurgical knife for treatment, such as incision and coagulation by applying high-frequency electric energy to a part to be treated 9B such as blood vessel that is pinched with a treatment part 32. The treatment tool 30 treats there with power that is wirelessly received at a power-reception part 39 (see FIG. 2), and so no cable is connected thereto for power supplying, meaning good operability.

The power-supply unit 20 to output AC power to the power transmission coil 11 of the trocar 10 includes a power supply 21 and a power transmission circuit 22. The power supply 21 outputs high-frequency and large power of 10 W to 100 W, for example. As illustrated in FIG. 2, a power transmission part 19 that generates AC magnetic field from the power supplied from the power supply 21 includes the power transmission coil 11, a power-transmission capacitor 15 and a power transmission circuit. The power transmission coil 11 is connected in series with the power-transmission capacitor 15, which make up a power-transmission side LC serial resonance circuit that generates AC magnetic field at a predetermined resonance frequency FR1. The power supply 21 outputs AC power at the resonance frequency FR1. Instead of the power-transmission capacitor 15, floating capacity of the power transmission coil 11 may be used in another configuration. The power transmission circuit 22 includes an impedance matching circuit (not illustrated) for impedance matching with the power supply 21 and the resonance circuit.

FIG. 1 and FIG. 2 illustrate the power-transmission capacitor 15 that is disposed in the trocar 10 and the power transmission circuit 22 that is disposed in the power-supply unit 20. Instead, the power-transmission capacitor 15 and the power transmission circuit 22 may be disposed in the trocar 10 or in the power-supply unit 20.

The switch 23 connected to the power-supply unit 20 may be a foot switch for ON/OFF control of the power output from the power-supply unit 20.

As described above, the treatment tool 30 has the power-reception part 39 including a power-reception coil 31 that is inductively coupled with the power transmission coil 11 of the power transmission part 19 and receives power wirelessly via the AC magnetic field. As illustrated in FIG. 2, the power-reception part 39 includes the power-reception coil 31, a power-reception capacitor 33 and a power-reception circuit 34. The power-reception coil 31 is connected in series with the power-reception capacitor 33, which make up a power-reception side LC serial resonance circuit that effectively receives AC magnetic field at a predetermined resonance frequency FR2. The resonance frequency FR2 of the power-reception side LC serial resonance circuit is substantially the same as the resonance frequency FR1 of the power-transmission side LC serial resonance circuit, and the operating system 1 performs wireless power |transfer |_{[M1]} effectively based on a magnetic resonance phenomenon. The resonance frequencies FR1 and FR2 may be selected appropriately in the range of 100 kHz to 20 MHz, from which a frequency that is allowed for use by law, e.g., 13.56 MHz is preferably selected.

Instead of the power-reception capacitor 33, floating capacity of the power-reception coil 31 may be used in another configuration. The power-reception circuit 34 rectifies an AC signal that the power-reception coil 31 receives into a DC signal, followed by smoothing, and then regulates it with a DC/DC converter to have voltage to be supplied to a driving part 35. The power-reception circuit 34 includes an impedance matching circuit (not illustrated) for impedance matching with the driving part 35 and the resonance circuit. The driving part 35 converts power from the power-reception circuit 34 into power suitable for driving at the treatment part 32. The treatment part 32 such as an electrosurgical knife receives a driving signal at the frequency of 350 kHz and at the voltage of a few hundreds Vpp from the driving part 35, for example.

The power-reception coil 31 that is of a long and thin solenoid type is disposed in the long and thin insertion part of the treatment tool 30 to be inserted into the subject 9 along the longer axis direction. The power-reception coil 31 that receives AC magnetic field generated from the power transmission coil 11 of the trocar 10 has a center axis that substantially agrees with the center axis of the insertion part. The power-reception coil 31 may have a length of 100 mm or more and 200 mm or less, for example, so as to allow a part thereof to be inserted always into the power transmission coil 11, which may have a length that is disposed over the total length of the insertion part. The power-reception coil 31 may be surrounded with insulating resin at the periphery, for example.

On the other hand, the power transmission coil 11 that is disposed inside of the housing 14 so as to be wound around the insertion hole 10H of the trocar 10 receives AC power and when generating AC magnetic field, generates heat due to Joule heat.

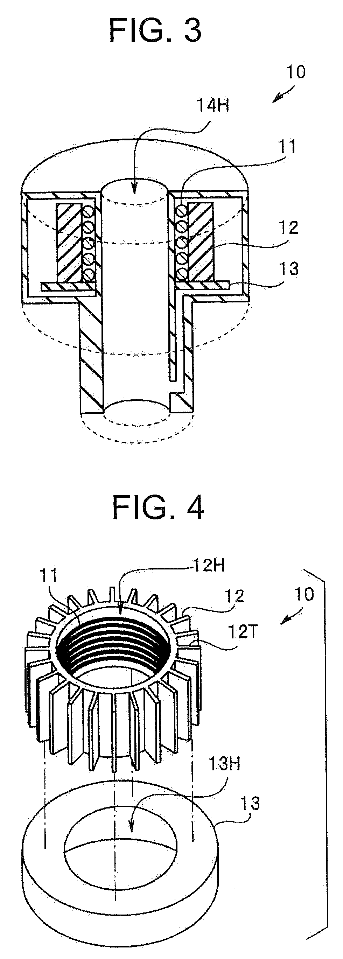

The heat dissipation member 12 that is a cylindrical member having a hollow inside transmits heat generated from the power transmission coil 11. This can prevent excessive temperature rise at the power transmission coil 11. The heat dissipation member 12 serving as a heat sink to which heat generated from the power transmission coil 11 is transmitted increases in temperature. As illustrated in FIG. 3 and FIG. 4, for example, the heat insulation member 13 is disposed under the heat dissipation member 12, i.e., on the side closer to the subject than the heat dissipation member 12, the heat insulation member having lower thermal conductivity than the heat dissipation member 12. The heat insulation member 13 is a cylindrical member that surrounds the lower face of the heat dissipation member 12 and has a hollow inside. That is, the heat dissipation member 12 and the heat insulation member 13 have a through hole 12H and a through hole 13H that are substantially the same in diameter, which make up an insertion opening of the trocar 10.

Even when the heat dissipation member 12 is heated, the heat insulation member 13 is disposed under the heat dissipation member 12, and so the temperature of the housing 14 on the lower side does not increase.

The heat dissipation member 12 preferably has thermal conductivity λ of 15 W/(m·K) or more. That is, the heat dissipation member 12 preferably is made of copper (λ=398 W/ (m·K)), silicon (λ=168 W/ (m·K)), aluminum nitride (λ=150 W/ (m·K)), iron (λ=84 W/ (m·K)), alumina (λ=32 W/ (m·K)), silicon nitride:Si₃N₄ (λ=27 W/(m·K)) or stainless steel (λ=17 W/ (m·K)), for example.

In order to prevent loss due to eddy current, the heat dissipation member 12 is preferably non-electrically conductive, and is made of a ceramic material, such as aluminum nitride particularly preferably.

When the heat dissipation member 12 is made of an electrically-conductive material, the power transmission coil 11 used has to include a core made of copper or the like that is surrounded with an insulating material. On the other hand, when the heat dissipation member 12 is made of a non-electrically conductive material, the power transmission coil may include a core without being surrounded with an insulating material.

The heat dissipation member 12 illustrated in FIG. 4, for example, has cooling fins 12T for better cooling efficiency by increasing the surface area. The cooling fin 12T may have a rod shape, for example, as long as it does not hinder the movement of air between fins. Warmed air moves naturally from down to up in the vicinity of the heat dissipation member 12 so as to generate convection of air in the housing, whereby heat at the heat dissipation member 12 is discharged to the outside. The housing 14 of the trocar 10 may have openings (not illustrated) for ventilation at the upper face and the side face. Air flowing from the opening at the side face into the housing is warmed by the heat dissipation member 12 and is discharged from the opening at the upper face.

Meanwhile, the heat insulation member 13 preferably has thermal conductivity λ of 0.3 W/(m·K) or less, and is 0.1 W/(m·K) or less particularly preferably. That is, the heat insulation member 13 preferably is made of resin material, such as epoxy resin (λ=0.21 W/(m·K)), silicone resin (λ=0.16 W/(m·K)), urethane resin (λ=0.034 W/(m·K)), silicon sponge (λ=0.08 W/(m·K)) or urethane foam (λ=0.029 W/(m·K)) made of such resin in a foamed shape, or glass wool (λ=0.045 W/(m·K)), for example. A space may be formed, and air (λ=0.024 W/(m·K)) may be used as the heat insulation member 13.

The heat dissipation member 12 of the trocar 10 does not increase in temperature excessively because of so-called natural cooling.

The power transmission coil 11 of the trocar 10 is not excessively heated due to the heat dissipation member 12 even when large power is applied to the power transmission coil 11. Since the heat insulation member 13 is disposed under the heat dissipation member 12 (on the subject side), temperature rise of the housing can be suppressed, especially at a part of the housing 14 that is in contact with the subject 9.

### <Modification example>

The following describes trocars 10A to 10F as modification examples of the trocar 10 of the first embodiment. All of the trocars 10A to 10F of the modification examples have the advantageous effects of the trocar 10 of the first embodiment, and have other additional effects.

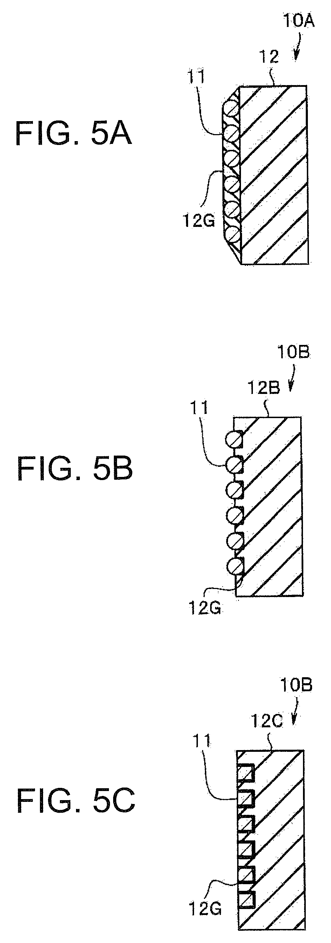

The trocar 10A as modification example 1 in FIG. 5A includes a high thermal conductivity material 12G such as thermal grease or a sheet made of a high thermal conductivity material so as to cover the power transmission coil 11. The thermal grease may contain silicone resin as a major component, to which metal particles such as silver are mixed for better thermal conductivity. The trocar 10A has good heat transmission efficiency.

The trocar 10B as modification example 2 in FIG. 5B includes a power transmission coil 11 disposed in a spiral-shaped groove at the inner periphery of a heat dissipation member 12B. The trocar 10B is configured to facilitate the disposition of the power transmission coil 11 in a predetermined shape at the inner periphery of the heat dissipation member 12.

The trocar 10C as modification example 3 in FIG. 5C includes a power transmission coil 11 having a core that is a rectangle in cross section, and the core is inserted into a rectangular groove of a heat dissipation member 12C. The trocar 10C enables more effective transmission of heat generated at the power transmission coil 11 to the heat dissipation member 12. The core of the power transmission coil 11 that is a circle in cross section may be inserted into a semicircular groove. That is, the heat dissipation member 12 having a groove that is fitted with the core of the power transmission coil 11 can widen the contact area between the power transmission coil 11 and the heat dissipation member 12 and so can improve heat transmission efficiency.

In the configuration of the core of the power transmission coil 11 inserted in the spiral-shaped groove of the heat dissipation member as well, the high thermal conductivity material 12G, e.g., thermal grease, is preferably disposed at a gap between the groove and the core.

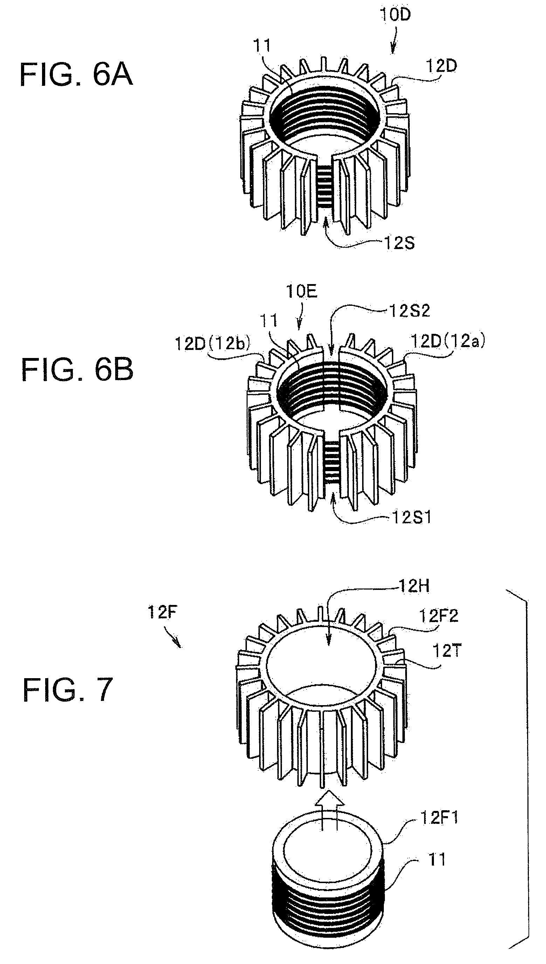

The trocar 10D as modification example 4 in FIG. 6A includes a heat dissipation member 12D made of an electrically-conductive metal material, in which a slit 12S is formed in the direction orthogonal to the circumferential direction. A metal material is easily processed and is not expensive compared with a ceramic material. The heat dissipation member made of an electrically-conductive member, however, generates eddy current flowing and generates loss when AC magnetic field is applied. The heat dissipation member 12D having the slit 12S therein, which has conductivity, can reduce such loss from eddy current. The slit 12S may be air gap or may be made of a non-electrically conductive member.

The trocar 10E as modification example 5 in FIG. 6B includes a heat dissipation member 12E made up of a plurality of metal members (electrically-conductive members) 12a and 12b that are electrically insulated by a plurality of slits 12S1 and 12S2.

The trocar 10F as modification example 5 in FIG. 7 includes a heat dissipation member 12F, including an inner tubular member 12F1 around which a power transmission coil 11 is wound, and an outer tubular member 12F2 joined to the outer periphery of the inner tubular member 12F1. Winding of the power transmission coil 11 around the outer periphery of the inner tubular member 12F1 is easier than winding at the inner periphery.

Further since the power transmission coil 11 of the trocar 10F is sandwiched between the inner tubular member 12F1 and the outer tubular member 12F2, heat generated at the power transmission coil 11 can be effectively transmitted to the heat dissipation member 12F. The inner tubular member 12F1 and the outer tubular member 12F2 may be made of the same material or different materials. At least one of the inner tubular member 12F1 and the outer tubular member 12F2 may be made of a high thermal conductivity material, and both of them are made of a high thermal conductivity material particularly preferably. Preferably the inner tubular member 12F1 and the outer tubular member 12F2 are in intimate contact via a high thermal conductivity material, such as thermal grease.

### <Second Embodiment>

The following describes an operating system 1G including a trocar 10G that is a second embodiment. Since the trocar 10G is similar to the trocar 10, the same reference numerals are assigned to common elements, and their descriptions are omitted.

While the heat dissipation member 12 in the trocar 10 is naturally cooled, the trocar 10G is forced-cooled. Then temperature rise at the housing of the trocar 10G can be suppressed as compared with the trocar 10, and the configuration described later has other additional advantageous effects.

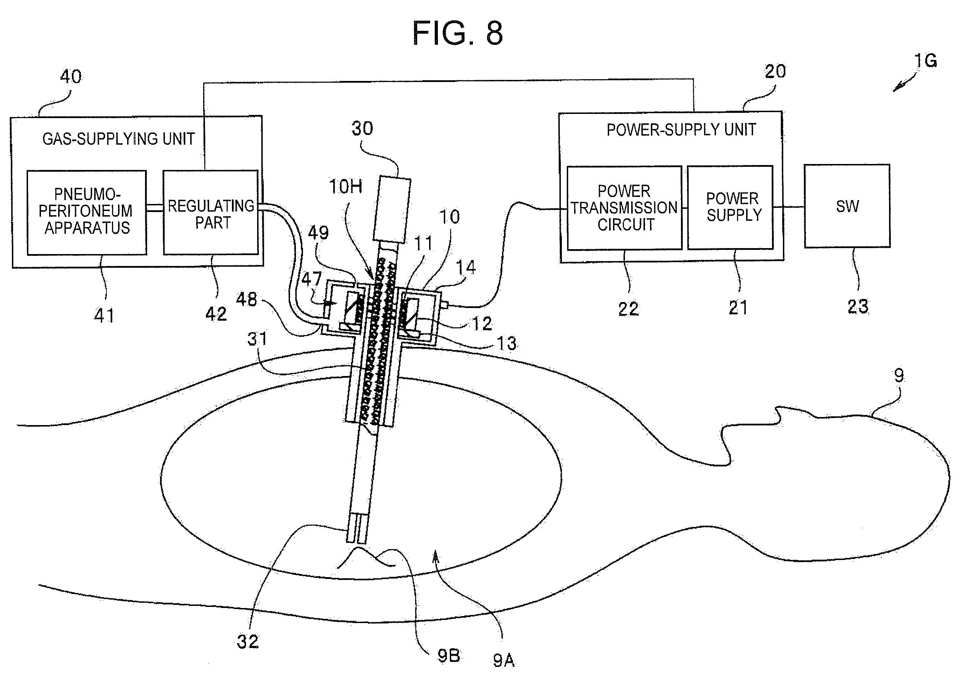

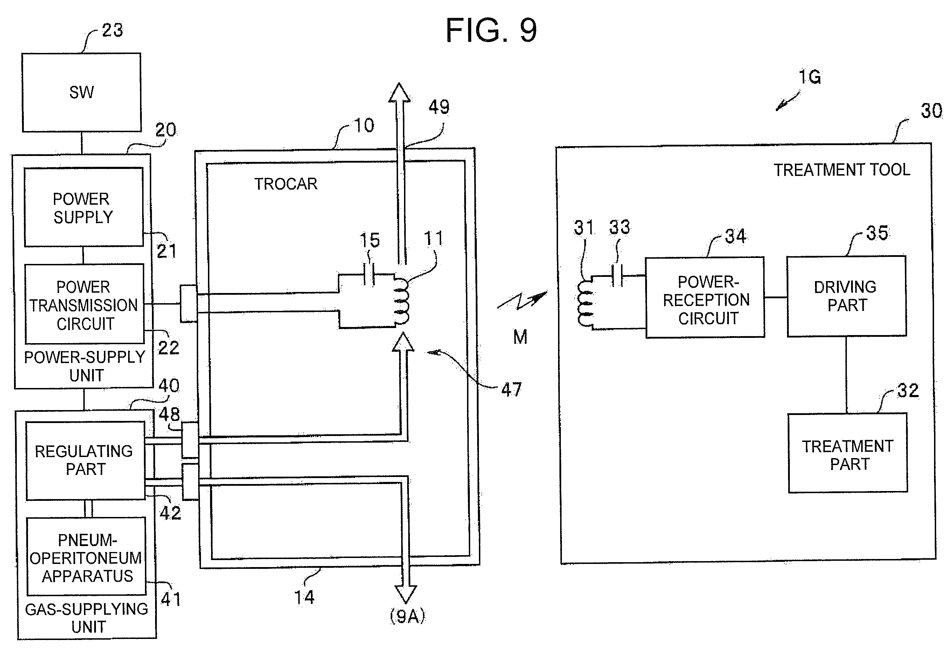

That is, as illustrated in FIG. 8 and FIG. 9, the operating system 1G includes a gas-supplying unit 40 to supply gas for forced-cooling of the heat dissipation member 12, in addition to the elements of the operating system 1.

A pneumoperitoneum apparatus 41 of the gas-supplying unit 40 fills an abdominal cavity to be treated with gas such as carbon dioxide to expand the abdominal cavity, thus keeping the viewing field for observation required to the treatment of the abdominal cavity and so allowing the operator to understand the treatment process sufficiently. Filling of gas into the abdominal cavity by the pneumoperitoneum apparatus is performed under the control of pressure of gas from a gas supplying source (not illustrated) such as a gas cylinder using a pressure-reducing device or a valve so as to keep the pressure in the abdominal cavity at the set pressure.

The trocar 10G includes an inlet 48 through which gas to cool the heat dissipation member 12 is introduced, a flow channel 47 including a pipe through which the gas flows, and an outlet 49 through which the gas is discharged. The inlet 48 is disposed at a lower part of the side face of the housing 14, and the outlet 49 is disposed at an upper part of the side face or at the upper face.

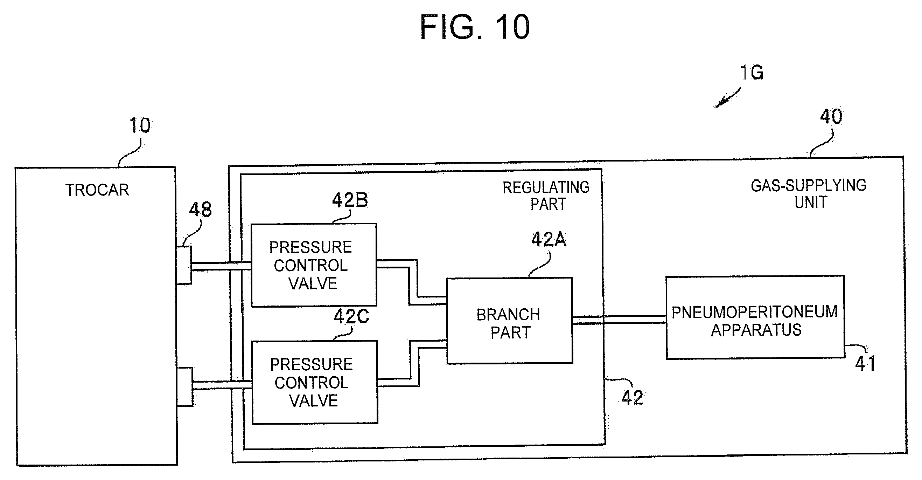

The gas-supplying unit 40 includes a regulating part 42 that divides gas supplied from the pneumoperitoneum apparatus 41 into a path to flow in the abdominal cavity and a path to flow in the flow channel 47 to cool the heat dissipation member 12. For instance, as illustrated in FIG. 10, the regulating part 42 includes a branch part 42A, a pressure control valve 42B that is controlled based on the pressure inside of the abdominal cavity and a pressure control valve 42C to control the flow rate of gas to flow in the flow channel 47.

The pressure control valves 42B and 42C may be passive one that regulates pressure on the secondary side based on balance between an elastic member such as a spring disposed internally and the pressure in the flow channel, or active one such as an electromagnetic valve. When the pressure control valve 42B is an active control valve, the pressure on the secondary side (abdominal cavity side) is detected by a pressure sensor (not illustrated) such as a diaphragm sensor, and the valve is controlled so that the measurement of pressure has a predetermined value. The pressure control valve 42C may have a configuration similar to that of the pressure control valve 42B, and is adjusted so that the pressure on the secondary side has a value required for flowing at the sufficient flow rate for coil cooling.

At least a part of the regulating part 42, e.g., the pressure control valve 42C may be disposed at the trocar 10, and the pressure control valve 42B may be disposed at the pneumoperitoneum apparatus 41.

The flow rate of gas supplied to the flow channel 47 may be set at a predetermined amount, and it is preferable that the regulating part 42 makes a flow-rate control part not illustrated regulate the flow rate or the like in accordance with the temperature or the amount of power of the power transmission coil 11 so as to prevent excessive supply of gas. Although the temperature of the power transmission coil 11 may be detected by a temperature sensor (not illustrated), it can be estimated from the amount of power applied to the power transmission coil 11 as well. That is, since the temperature and the amount of power of the power transmission coil 11 have a proportional relationship, regulation based on the amount of power has the same meaning as the regulation based on the temperature of the power transmission coil.

For instance, control is performed so that gas is supplied to the flow channel 47 only when the temperature or the amount of power of the power transmission coil 11 is a predetermined value or more. Alternatively the flow rate of gas to be supplied to the flow channel 47 may be controlled so as to be in proportion to the temperature or the amount of power of the power transmission coil 11.

The regulating part 42, which regulates the pressure control valve 42C based on information on the temperature of the power transmission coil 11 or on the amount of power from the power-supply unit 20, can improve the efficiency of gas supplying and so is economical.

In another configuration, gas subjected to regulation by the pressure control valve 42B may be supplied to the abdominal cavity via the flow channel 47. The operating system configured to cool the power transmission coil 11 with gas for pneumoperitoneum is simpler than the configuration of the operating system 1G. That is, it does not have to include the branch part 42A and the pressure control valve 42C.

An apparatus exclusively used to supply fluid to cool the heat dissipation member may be provided. In this case, the fluid used may be liquid such as water.

The present invention is not limited to the above-described embodiments, and can be changed and modified variously without changing the gist of the present invention.

This application claims the benefit of priority to JP Patent Application No. 2013-172474 filed in Japan on August 22, 2013 based thereon, the disclosure of which is herein incorporated by reference in the specification, claims and drawings of this application.

## Claims

1. A trocar, comprising:
a housing;
an insertion tube that is integral with the housing and is inserted into a subject;
a power transmission coil that is disposed inside of the housing and generates AC magnetic field to be applied to an insertion hole into which a treatment tool is to be inserted;
a heat dissipation member that transmits heat generated from the power transmission coil; and
a heat insulation member disposed on a side of the subject with reference to the heat dissipation member and having lower thermal conductivity than the heat dissipation member.

2. The trocar according to claim 1, wherein the heat dissipation member has a circumferential direction and includes an electrically-conductive member having a slit therein in a direction orthogonal to the circumferential direction.

3. The trocar according to claim 2, wherein the heat dissipation member includes a plurality of electrically-conductive members that are electrically insulated by a plurality of slits.

4. The trocar according to claim 2, wherein the heat dissipation member includes a cylindrical member having an inner periphery on which the power transmission coil is wound.

5. The trocar according to claim 4, wherein the heat dissipation member includes:
an inner tubular member having an outer periphery around which the power transmission coil is wound; and
an outer tubular member joined to the outer periphery of the inner tubular member.

6. The trocar according to claim 4 or 5, wherein the heat dissipation member has a spiral-shaped groove, in which the power transmission coil is inserted.

7. The trocar according to any one of claims 1 to 6, further comprising:
an inlet connecting part through which fluid to cool the heat dissipation member is introduced;
a flow channel through which the fluid flows; and
an outlet connecting part through which the fluid is discharged.

8. The trocar according to claim 7, wherein the fluid is gas supplied from a pneumoperitoneum apparatus.

9. The trocar according to claim 8, wherein the gas is supplied into an abdominal cavity of a subject via the flow channel.

10. The trocar according to claim 8, wherein the gas to be supplied to the flow channel is regulated in accordance with temperature or an amount of power at the power transmission coil.

11. The trocar according to claim 10, wherein when the temperature or the amount of power at the power transmission coil is a predetermined value or more, the gas is supplied to the flow channel.
